Europäisches Patentamt

European Patent Office

Office européen des brevets

(11) Publication number: **0 382 215**
**A1**

# EUROPEAN PATENT APPLICATION

(21) Application number: 90102492.7

(51) Int. Cl.⁵: **B01J 20/26, B01J 20/32**

(22) Date of filing: 08.02.90

(30) Priority: 08.02.89 JP 27510/89

(43) Date of publication of application:
16.08.90 Bulletin 90/33

(84) Designated Contracting States:
DE FR GB

(71) Applicant: MITSUI TOATSU CHEMICALS INC.
No. 2-5, Kasumigaseki 3-chome
Chiyoda-Ku Tokyo 100(JP)

(72) Inventor: Hitachi, Kiyoshi, 17-33-202, Katakura
1-chome,
Kanagawa-ku, Yokohama-shi,
Kanagawa-ken,(JP)
Inventor: Murata, Naohiro,
5-45, Dai 4-chome, Kamakura-shi,
Kanagawa-ken,(JP)
Inventor: Fujikake Shiro,
2089-5, Iijima-cho, Sakae-ku,
Yokohama-shi, Kanagawa-ken,(JP)
Inventor: Ohkawa, Kouhei,
386-1 Hisasue, Takatsu-ku,
Kawasaki-shi, Kanagawa-ken,(JP)
Inventor: Nakabayashi, Nobuo,
6-20, Koganehara 5-chome,
Matsudo-shi, Chiba-ken,(JP)
Inventor: Koshikawa, Syozo,
8-3, Higashigaoka 2-chome,
Meguro-ku, Tokyo,(JP)

(74) Representative: Vossius & Partner
Siebertstrasse 4 P.O. Box 86 07 67
D-8000 München 86(DE)

(54) Porous adsorbent for beta-2-Microglobulin.

(57) A porous adsorbent for $\beta_2$-microglobulin made of a polyamino acid having a porous surface. This adsorbent is highly selective for $\beta_2$-microglobulin without adsorbing useful proteins other than $\beta_2$-microglobulin and is a good and rapid $\beta_2$-microglobulin adsorber.

## POROUS ADSORBENT FOR $\beta_2$-MICROGLOBULIN

The present invention relates to an adsorbent for $\beta_2$-microglobulin, and more particularly to an adsorbent for removing $\beta_2$-microglobulin present in blood or blood plasma.

$\beta_2$-Microglobulin is the light chain (L chain) of proteins containing two chains and constituting histocompatibility antigens (in the case of human beings, HLA, class 1), and it is found on nearly all cell surfaces, as well as in liquids in the free state, where it is not bound to heavy chains (H chains).

It is known from analysis of the total amino acid sequence that $\beta_2$-microglobulin is a simple protein that is similar to the C domains of immunoglobulins that it has a molecular weight of about 12,000, and that it does not have sugar chains.

In a patient who has been subjected to hemodialysis for a long period, the concentration of free $\beta_2$-microglobulin in the blood increases to 10 to 100 times that of healthy people, and it is believed that $\beta_2$-microglobulin causes the carpal tunnel syndrome (amyloidosis) that occurs at a high rate in patients undergoing dialysis.

Although attempts have been made to remove $\beta_2$-microglobulin from the blood or blood plasma, practical techniques have not yet been found. For instance, its separation by a dialysis membrane also removes other useful proteins in addition to $\beta_2$-microglobulin, and the removal of $\beta_2$-microglobulin is insufficient.

Adsorbents, such as an antibody to B$_2$-microglobulin immobilized on an inert carrier are disclosed in JP-A-504911/1986. However, the preparation of such adsorbents including the production and cloning of the antibodies is time consuming and expensive. Therefore, said adsorbents are not practical for efficiently removing $\beta_2$-microglobulin from the blood or blood plasma.

In cases wherein a highly hydrophobic ligand bound to various porous carriers effects the adsorption (see JP-A-99875/1988 and 240068/1987), although the adsorption of $\beta_2$-microglobulin is high due to the ligand's strong hydrophobic nature, other proteins (particularly highly hydrophobic proteins, such as immunoglobulins), lipids, etc., which are present in large amounts in blood or blood plasma, are adsorbed as well. Therefore, it is considered that this technique is difficult to use, since in actual blood or blood plasma the adsorbed amount and the adsorption speed of $\beta_2$-microglobulin are lower.

Further, in the carriers disclosed in the Japanese patent applications noted above, the range of the pore size, of from 20 to 2,000 Å, and the range of the molecular weight, of from 10,000 to 600,000 are considered to be unspecific for selecting $\beta_2$-microglobulin by size over other proteins.

Further, the adsorbents of these prior proposals are not biocompatible, and if they are practically applied as they are to blood and blood plasma, the problem of blood compatibility remains.

Blood compatibility may be enhanced, in particular by using an antithrombotic agent or by coating the adsorbent with a polymer such as polyhydroxyethyl methacrylate. However, there is a concern that this simple coating will dissolve or separate from the adsorbent during use.

Therefore, the coating material should be bound to the adsorbent by coordinate bonds.

Thus, the technical problem underlying the present invention is to provide an efficient adsorbent that selectively adsorbs $\beta_2$-microglobulin in blood or blood plasma and thus allows its reliable removal therefrom.

A further technical problem underlying the present invention is to provide an efficient process for selectively removing $\beta_2$-microglobulin from a liquid containing it, including blood or blood plasma, wherein said porous adsorbents are used.

The solution of the above technical problems is based on the finding that polyamino acids, even if they are chemically synthesized, are biocompatible, since the main chain has a polypeptide structure that is present in proteins. It has now been determined that, in polyamino acids, unique secondary-structure (conformation) changes (randoms, $\alpha$-helices, and $\beta$-sheets) occur due to the polypeptide structure itself. These structural changes result in a change in the hydrophobic nature of the synthesized polyamino acids, and the resulting hydrophobic nature makes the adsorption different with respect to individual proteins, thereby enabling various proteins to be separated.

By controlling the range of the pore size of the porous body containing a polyamino acid, the type of molecules that diffuse into the interior of the beads can be restricted, thereby enabling only certain proteins present in the blood or blood plasma to be selectively adsorbed and providing a practical and efficient means to selectively adsorb $\beta_2$-microglobulin.

The subject matter of the present invention therefore is a porous adsorbent for $\beta_2$-microglobulin comprising a polyamino acid having a porous surface.

Preferably, the pore size distribution of the porous adsorbent has a peak in the range of about 9,000 to about 70,000 in terms of the molecular weight of dextrans according to gel permeation chromatography (GPC) for a dextran standard molecular weight substance.

A further subject matter of the invention is a process for selectively removing $\beta_2$-microglobulin from a liquid containing it, including blood or blood plasma, wherein said porous adsorbent is used. The process is carried out outside of the human body.

Fig. 1 is a diagram showing the pore size distribution of the porous adsorbent obtained in Example 1.

Fig. 2 is a diagram showing the pore size distribution of the porous adsorbent obtained in Comparative Example 1.

Fig. 3 is an explanatory view of the apparatus used in the $\beta_2$-microglobulin adsorption test as described below.

Polyamino acids used in the present invention include homopolyamino acids, such as polyalanine, polycystine, polyglycine, polyoxyproline, polyisoleucine, polyleucine, polymethionine, polyphenylalanine, polyproline, polyserine, polythreonine, polytryptophan, polytyrosine, polyvaline, polyarginine, polylysine, polyhistidine, polyglutamic acid, and polyaspartic acid, and their derivatives, or copolymers of two or more monomers of these homopolyamino acids.

In the present invention the selective adsorption of $\beta_2$-microglobulin can be optimized by controlling the hydrophobic nature of the porous adsorbent containing the polyamino acid. The hydrophobic nature due to the primary structure of polyamino acids can be controlled by introducing suitable substituents to the polyamino acid side chains. Although there is no particular limitation on the controlling method, for example the control can be effected by esterification of the side chain carboxyl groups of polyaspartic acid or polyglutamic acid with an alkyl group, such as a methyl or ethyl group or with an aromatic residue, such as a benzyl or naphthyl group, or by reacting the side chain amino groups of polylysine, polyhistidine or polytryptophan with an acid chloride or with an isocyanate having a hydrophobic group, such as dodecanoyl chloride, stearoyl acid chloride, or benzoyl chloride, or by reacting side chain hydroxyl groups of the amino acid or polyamino acid with an isocyanate having a hydrophobic group, such as phenyl isocyanate, n-butyl isocyanate, and napthyl isocyanate.

In a similar manner, hydrophobic groups can be introduced into the side chain hydroxyl groups of polyoxyproline, polyserine, polythreonine and polytyrosine . The hydrophobic groups may be introduced after the polymerization of the amino acids as described above, or they may first be introduced into the amino acids in a suitable manner, and then polymerization may be effected to obtain the desired polymer.

The regulation of the hydrophobic nature can be effected by changing the type of the hydrophobic group(s) to be introduced, by changing the rate of the introduction of the hydrophobic group, or by using a combination thereof.

If the polymerization of the amino acid is effected before introduction of the hydrophobic group, the hydrophobic nature can be regulated in a suitable manner, for example by hydrolyzing the polymeric product to partially remove the hydrophobic group.

The porous adsorbent comprising a polyamino acid having a porous surface of the present invention may be used as such after forming the above-mentioned polyamino acid itself into a porous membrane, porous fibers, or porous beads. According to another embodiment, the polyamino acid may also be coated or chemically bound to various carriers followed by making the carrier porous, or it may be coated or chemically bound to a porous carrier.

The carriers used herein may be made of inorganic substances such as glass, metal salts, and carbon, or organic substances, such as polymers. The carriers may take various physical shapes such as beads, membranes, fibers or hollow yarns (hollow fibers).

The porosity can be selected arbitrarily, such that the rate of voids (pores) preferably is in the range of 10 to 99 %, and more preferably is in the range of 50 to 95 %. The rate of void is defined by the rate of void into which $D_2O$ can diffuse to the whole volume of the adsorbent.

The coating or chemically bonding of the polyamino acids to various carriers can be conducted in such a way that, for example, a desired carrier, such as glass beads, cellulose fibers, nylon fibers, activated charcoal, hydroxyapatite, polyvinyl chloride sheet, polystyrene beads, or nylon hollow yarn, is immersed in a solution of the polyamino acid thereby impregnating it with the polyamino acid. The excess polyamino acid solution is removed, for example by filtration, and the solvent is evaporated, or a poor solvent for the polyamino acid is added.

The polyamino acids are chemically bound to the carrier using functional groups of the carrier and a suitable bonding reagent. For example, a bifunctional reagent, such as toluene diisocyanate or terephthaloyl chloride, is reacted with aminopropyl silica gel, thereby binding the amino groups of the polyamino acid into the aminopropyl silica gel.

The polyamino acid itself may also be formed into beads, for example, by dissolving the polyamino acid in a suitable solvent, then dispersing the solution into a medium incompatible with the solvent. The solvent is gradually evaporated and removed, thereby depositing the polyamino acid as spheres (JP-A-1728/1987).

Also there is a method in which an amino acid NCA (N-carboxyamino acid anhydride), which has not yet been polymerized, is polymerized in a solvent compatible with the amino acid NCA but incompatible with the polymerized amino acid NCA.

In the former method, a poor solvent for the polyamino acid is added to form beads, and the solvent is removed, for example by extraction, thereby enabling the beads to be porous, the pore size being controllable by the type and the amount of the poor solvent used. In the latter method, the pore size can be controlled by the type and temperature of the solvent used in the polymerization, the polymerization catalyst, and by the polymerization time.

The polyamino acids which can be formed into beads by the former method include polyalanine, polyaspartic acid, polyglutamic acid, polyglycine, polyoxyproline, polyisoleucine, polyleucine, polyproline, polyserine, polythreonine, polyvaline, and their derivatives, and copolymers of two or more of these.

In the latter method, basically, although in theory any amino acid or amino acid derivative may be treated, in order to control the pore size it is necessary to control the type of solvent, the polymerization temperature, the polymerization catalyst, and the polymerization time.

Although the size of the beads obtained in these methods can arbitrarily be varied, it is preferably within the range of 0.1 μm to 5 mm, and more preferably the size of the beads is 50 μm or greater, in view of problems including pressure loss of the system, which may arise while blood or blood plasma is practically passed through the beads to remove $\beta_2$-microglobulin.

Beads made, for example of a porous glass, a porous silica gel, or a porous polymer carrier, are immersed in a polyamino acid solution, and then the solvent may be removed, to coat the porous carrier surface with a polyamino acid membrane. In this case, since the pore size is dependent on the pore size of the carrier, it is preferable to use a carrier whose pore size is controlled.

Further, the polyamino acids may be bound to the porous carrier using functional groups present on the carrier such as amino, carboxyl, epoxy, hydroxyl, acid chloride, isocyanate or chloromethyl groups.

For example, the amino acid NCA may be polymerized with the amino groups of porous aminopropyl silica gel used as starting material, or a polyamino acid having side chain amino groups or a terminal amino group may be condensed onto polyacrylic acid porous beads in the presence of a suitable dehydrating catalyst.

The pore size of the porous beads comprising a polyamino acid obtained as described above can be assessed by carrying out a GPC measurement using a column filled with the beads.

In this procedure the porous beads comprising the polyamino acid are sieved in a suitable manner, and are placed in a column for liquid chromatography.

Preferably the size of the beads to be assessed is 100 μm or below; the bead should be as uniform as possible. The column used is conveniently a commercially available stainless steel column having e.g. an inner diameter of 4.6 mm and a length of 150 mm manufactured by Nippon Seimitsu Co., Ltd.

The standard molecular weight substance for the GPC measurement is desirably one that is as low as possible in interacting with the polyamino acid, and a dextran standard molecular weight substance (commercially available and manufactured by Sigma Chemical Co.) is considered appropriate. Other standard molecular weight substances, such as spherical protein and polyethylene glycol standard substances, may interact with polyamino acids and do not dissolve out necessarily according to their molecular weight, or sometimes they are adsorbed, so that they are not appropriate.

The measurement method may be the common water-type GPC measurement using standard molecular weight dextrans. In this procedure distilled water is used as eluent, and the elution volumes for dextrans having various molecular weights are measured. The results of the measurement can be assessed by preparing a calibration curve representing the relationship between the molecular weights and the elution volumes. That is, the molecular weights of the eluted dextrans are plotted along the ordinate axis, and the rates of the elution volumes to the whole volume of the column are plotted along the abscissa axis. Accordingly, the exclusion limit molecular weight and the overall permeation limit molecular weight of the polyamino acid beads can be determined and the pore sizes in terms of the dextrans can be calculated. The pore size distribution can be assessed relatively by approximating the obtained calibration curve by the cubic Spline function, and by calculating the rate of change in the elution volume against the change in molecular weight.

That is, the common logarithm of the molecular weight of 2,000,000 to 20 is changed by every 0.1, the elution volumes are calculated by the above approximate expression, and the change rate α of the elution volume to the molecular weight is computed according to the equation:

4

$$\alpha = (V_{i+1} - V_i)/(100 - V_0)$$

where $V_i$ represents the ratio of the eluted volume to the overall column volume, and $V_0$ represents the ratio (%) of the adsorbent apparent volume (void volume of the adsorbent-filled column) to the overall column volume.

When the rate of change $\alpha$ is plotted against the logarithm of the molecular weight, the relative pore size distribution can be obtained.

The porous adsorbent for adsorbing $\beta_2$-microglobulin selectively from the blood or blood plasma is one in which the pore size distribution has a peak in the range of about 9,000 to about 70,000, preferably in the range of 10,000 to 40,000 expressed in terms of the molecular weight of the dextrans used as a standard molecular weight substance. Adsorbants in which the peak is in the molecular weight range more than about 70,000, or in the molecular weight range less than 9,000, cannot provide good selective adsorption and are thus not recommended.

The hydrophobic nature of the surface of the porous adsorbent comprising a polyamino acid can be assessed by measuring the eluted volumes of alcohols of different hydrophobic nature in the same system as that for the above GPC assessment, and finding the ratios of the eluted volumes.

That is, the eluted volumes of methanol and an alcohol having a linear alkyl group, such as methanol, ethanol, n-propanol, n-butanol, and n-pentanol, are measured, and the ratios of the eluted volumes to the eluted volume of methanol are computed. The values obtained by dividing the eluted volume of n-butanol to the eluted volume of methanol (k value) are compared.

The greater the k value, the stronger the hydrophobic nature of the porous adsorbent, and the smaller the value, the weaker the hydrophobic nature as expressed by the equation:

$$k = V_{Bu}/V_{Me}$$

where $V_{Bu}$ represents the eluted volume of n-butanol, and $V_{Me}$ represents the eluted volume of methanol.

In the present invention the k value is generally 1.3 or higher, preferably 1.3 ~ 7.0, more preferably 1.5 ~ 3.0.

The present invention provides a convenient and effective technique for selectively adsorbing and removing $\beta_2$-microglobulin from the blood or blood plasma. Although the mechanism of this adsorption has not yet been clarified and we do not wish to be bound by any particular theory or suggested mode of operation, it is believed that by controlling the pore size of a porous adsorbent containing a polyamino acid, separation can be effected in accordance with the size of the molecules to be separated, and also by controlling the hydrophobic nature of the polyamino acid component, $\beta_2$-microglobulin is selectively adsorbed.

The present porous adsorbent for $B_2$-microglobulin exhibits such excellent effects that it (1) is high in selectivity without adsorbing useful proteins other than $\beta_2$-microglobulin, (2) achieves rapid adsorption speed and amounts of $\beta_2$-microglobulin, and (3) is biocompatible.

The present invention will now be described in more detail with reference to Examples, although the present invention is not limited to the Examples. An adsorption test of $\beta_2$-microglobulin is described later.

Example 1

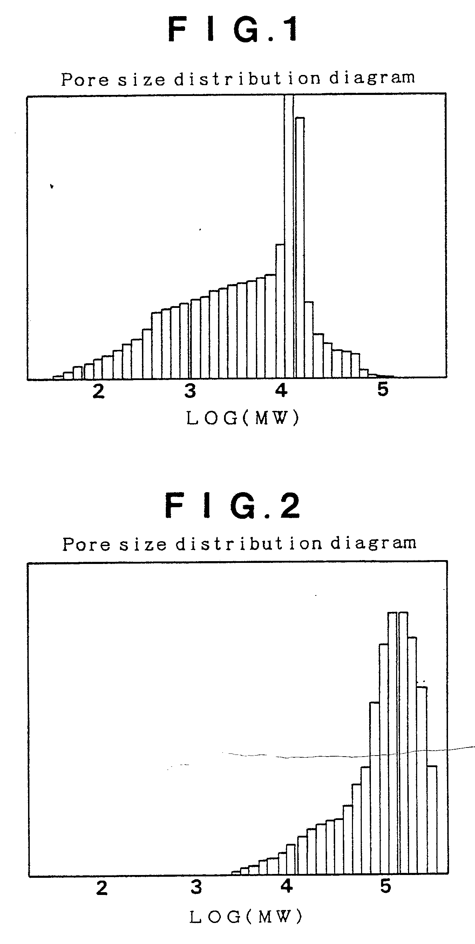

5 g of poly-$\gamma$-methyl-L-glutamate (hereinafter abbreviated PMLG) having an average polymerization degree of about 700 was dissolved in ethane dichloride, to prepare a 2.5 wt.% solution of PMLG. 15 ml of methyl laurate was added to the solution, and it was stirred and dispersed into a 1 wt.% aqueous solution of partially saponified polyvinyl alcohol. At 50°C, the ethane dichloride was evaporated and removed, the resulting beads were washed with water, and the methyl laurate was eluted and removed completely using methanol. The thus obtained PMLG beads were seaved and classified into two groups of 40 to 100 $\mu$m beads and 100 to 200 $\mu$m beads. The 40 $\mu$m to 100 $\mu$m beads were placed in a column having an inner diameter of 4.6 mm and a length of 15 cm to fill the column. Using water as an eluent, a GPC measurement of a dextran standard molecular weight substance (manufactured by Sigma Chemical Co.) was carried out. Based on the results of the measurement, the pore size distribution was determined, which is shown in Fig. 1, and it can be understood that the peak of the distribution lies in the range of 9,000 to 40,000 in terms of the molecular weight of the dextran . The k value that is the ratio of the elution volume of n-butanol to that of methanol was 1.31.

Comparative Example 1

Example 1 was repeated, except that, instead of methyl laurate, 20 mℓ of dioctyl phthalate was added, thereby preparing porous beads. The results of the GPC assessment of the beads are shown in Fig. 2. The k value of the beads was 1.02.

Example 2

PMLG having an average polymerization degree of about 2,000 was subjected to interesterification in benzyl alcohol for 6 hours at its reflux temperature. The elemental analysis of the partially benzylated PMLG was as shown in Table 1.

The results show that about 26 % of the amino residue of the PMLG was benzylated.

Table 1

|  | C | H | N (%) |
|---|---|---|---|
| Before reaction | 50.3 | 6.3 | 9.8 |
| After reaction | 55.7 | 6.2 | 8.6 |

0.5 g of this partially benzylated PMLG was dissolved in 10 g of ethane dichloride containing 1.5 mℓ of methyl laurate, 100 g of glass beads having a diameter of 0.5 mm were immersed in the solution, then after the excess polyamino acid solution was filtered off, they were dried spontaneously at about 40° C. Next the thus coated beads were immersed into methanol to completely extract the methyl laurate. The pore size of the thus obtained beads was assessed in the same way as in Example 1, and it was found that the peak of the pore size distribution was at about 10,000. The k value thereof was 1.51.

Comparative Example 2

Example 2 was repeated, except that methyl laurate was not added, thereby preparing beads. The peak of the pore size distribution of the beads was at 5,000 or below. The k value thereof was 1.49.

Example 3

5 g of γ-benzyl-L-glutamic acid was dispersed and suspended into 100 mℓ of THF by stirring well. While the dispersion was stirred, phosgene was blown into the dispersion until the dispersion became completely clear, then the supply of the phosgene was stopped and nitrogen gas was blown into the solution for about 30 min. The THF was removed outside the system using an evaporator, the remainder was dissolved again in ethyl acetate, excess n-hexane was added thereto, the mixture was cooled for 1 hour at -10° C, and then the deposited γ-benzyl-L-glutamic acid NCA crystals were filtered and dried.

Additionally, 5 g of L-leucine was processed in the same way as above to produce NCA crystals. 2.63 g of the thus obtained γ-benzyl-L-glutamic acid NCA and 0.78 g of the thus obtained L-leucine NCA were dissolved in 350 g of ethane dichloride, then 0.1 g of triethylamine was added to the solution and polymerization was effected for 6 hours at 30° C.

Part of the polymeric liquid was taken out and was dropped into methanol. Elemental analysis of the deposited polymer was carried out, the results being shown in Table 2.

Table 2

| C | H | N (%) |
|---|---|---|
| 65.3 | 6.7 | 7.7 |

From the results it can be understood that of the amino residues of the polyamino acids, leucine was 34 % and γ-benzyl-L-glutamic acid was 66 %.

To 100 g of the polymeric liquid were added 100 g of chloroform and 2 mℓ of methyl laurate followed by stirring well, then 5 g of cellulose fibers (adsorbent cotton) was immersed therein, the excess polyamino acid solution was removed by filtering under suction, then after the remainder was dried spontaneously at room temperature, it was immersed in methanol, and the solvent was removed completely.

The thus obtained porous fibers were placed in a stainless column having an inner diameter of 4.6 mm and a length of 5 cm filling the column, and the GPC assessment was carried out in the same way as in Example 1. The pore size distribution had a peak at 20,000, and the k value was 1.44.

Comparative Example 3

Example 3 was repeated, except that 5 mℓ of linolic acid was added to 100 g of the polymeric liquid obtained in Example 3, thereby preparing porous fibers. The pore size distribution had a peak at about 20,000, and the k value was 1.18.

Adsorption Test

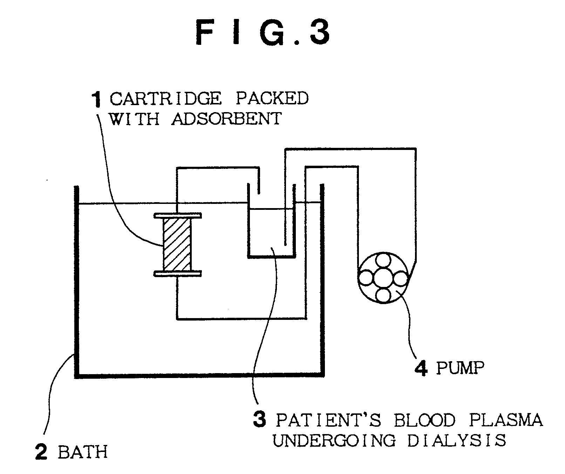

With respect to the adsorbents obtained in the above examples and comparative examples, a B₂-microglobulin adsorption test was carried out using the apparatus shown in Fig. 3. In Fig. 3, reference numeral 1 indicates a cartridge packed with the adsorbent of the present invention, 2 indicates a 37°C thermostat liquid bath, 3 indicates the blood plasma of a patient undergoing dialysis, and 4 indicates a pump. The cartridge may be a Tigon-tube R-3603 (trade name, manufactured by Norton Co.) having an inner diameter of 4.7 mm loaded with the adsorbent to be tested. 50 mℓ of blood plasma of a patient undergoing artificial dialysis was circulated at 37°C and a flow rate of 50 mℓ/hr by using the pump, and the change of the concentration of $\beta_2$-microglobulin in the blood plasma with time was measured.

The results are shown in Table 3.

EP 0 382 215 A1

Table 3

| Adsorbent Composition of plasma | Example 1 | | | Example 2 | | | Example 3 | | | Comparative Example 1 | | | Comparative Example 2 | | | Comparative Example 3 | | |
|---|---|---|---|---|---|---|---|---|---|---|---|---|---|---|---|---|---|---|
| Time (hr.) | A | B | C | A | B | C | A | B | C | A | B | C | A | B | C | A | B | C |
| 0 | 9.8 | 4.7 | 1.3 | 7.5 | 4.6 | 1.3 | 7.9 | 4.7 | 1.3 | 9.8 | 4.7 | 1.3 | 7.5 | 4.6 | 1.3 | 7.9 | 4.7 | 1.3 |
| 0.5 | 6.8 | 4.7 | 1.3 | 5.2 | 4.6 | 1.3 | 5.4 | 4.7 | 1.3 | 8.1 | 4.7 | 1.3 | 5.5 | 4.6 | 1.3 | 7.1 | 4.7 | 1.3 |
| 1.0 | 4.9 | 4.7 | 1.3 | 2.9 | 4.6 | 1.3 | 3.1 | 4.7 | 1.3 | 7.9 | 4.7 | 1.3 | 5.4 | 4.6 | 1.3 | 7.3 | 4.7 | 1.3 |
| 2.0 | 2.7 | 4.7 | 1.3 | 1.4 | 4.6 | 1.3 | 1.5 | 4.7 | 1.3 | 7.6 | 4.7 | 1.3 | 4.6 | 4.6 | 1.3 | 6.8 | 4.7 | 1.3 |
| 3.0 | 1.0 | 4.7 | 1.3 | <0.25 | 4.6 | 1.3 | <0.25 | 4.7 | 1.3 | 6.7 | 4.7 | 1.3 | 4.8 | 4.6 | 1.3 | 6.2 | 4.7 | 1.3 |
| 4.0 | <0.25 | 4.7 | 1.3 | <0.25 | 4.6 | 1.3 | <0.25 | 4.7 | 1.3 | 6.1 | 4.7 | 1.3 | 4.6 | 4.6 | 1.3 | 6.0 | 4.7 | 1.3 |

*Note    A : Concentration of $\beta_2$-microglobulin (mol/$\ell$)

B : Total amount of proteins (g/d$\ell$)

C : Ratio of albumin/globulin

**Claims**

1. A porous adsorbent for $\beta_2$-microglobulin which comprises a polyamino acid having a porous surface.

2. The porous adsorbent as claimed in claim 1, wherein the pore size distribution of the porous adsorbent has a peak in the range of about 9,000 to about 70,000 in terms of the molecular weight of the dextrans used as a standard molecular weight substance in gel permeation chromatography (GPC).

3. The porous adsorbent as claimed in claim 1 or 2 wherein the polyamino acid is selected from polyalanine, polycystine, polyglycine, polyoxyproline, polyisoleucine, polyleucine, polymethionine, polyphenylalanine, polyproline, polyserine, polythreonine, polytryptophan, polytyrosine, polyvaline, polyarginine, polylysine, polyhistidine, polyglutamic acid, polyaspartic acid, and their derivatives, or copolymers of two or more thereof.

4. The porous adsorbent as claimed in any one of claims 1 to 3 wherein the selective adsorption of $\beta_2$-microglobulin is controlled by controlling the hydrophobic nature (k-value) of the polyamino acid.

5. The porous adsorbent as claimed in claim 4, wherein the hydrophobic nature of the adsorbent is controlled by introducing a substituent to the polyamino acid side chains.

6. The porous adsorbent as claimed in claim 5, wherein the substituent is introduced after polymerization of the amino acid.

7. The porous adsorbent as claimed in claim 5, wherein the substituent is introduced before polymerization of the amino acid.

8. The porous adsorbent as claimed in claims 6 or 7 wherein for the introduction of a hydrophobic group a side chain carboxyl group of the amino acid or polyamino acid is esterified with an alkyl group or an aromatic group, a side chain amino group is reacted with an acid chloride or with an isocyanate having a hydrophobic group, or a side chain hydroxyl group of the amino acid or polyamino acid is reacted with an isocyanate having a hydrophobic group.

9. The porous adsorbent as claimed in any one of claims 1 to 8 wherein the adsorbent has a hydrophobic value k of at least 1.3.

10. The adsorbent as claimed in claim 1, wherein the polyamino acid itself is porous.

11. The adsorbent as claimed in claim 10, wherein the adsorbent comprises a porous membrane made of the polyamino acid.

12. The adsorbent as claimed in claim 10, wherein the adsorbent comprises porous fibers made of the polyamino acid.

13. The adsorbent as claimed in claim 10, wherein the adsorbent comprises porous beads made of the polyamino acid.

14. The adsorbent as claimed in claim 1, wherein the adsorbent comprises the polyamino acid coated on or chemically bound to a carrier.

15. The adsorbent as claimed in claim 14, wherein the carrier is a porous carrier.

16. The adsorbent as claimed in claim 1, wherein the adsorbent comprises a polyamino acid chemically bound onto a carrier utilizing functional group present on the carrier and a bonding reagent.

17. The adsorbent as claimed in claim 14, wherein the carrier is in the shape of beads, a membrane, fiber, or hollow yarn.

18. The adsorbent as claimed in any one of claims 1 to 17, wherein the porosity of the adsorbent is in the range of 10 to 99 %.

19. The adsorbent as claimed in any one of claims 1, 10 and 14 wherein the adsorbent is a porous bead having a diameter in the range of 0.1 μm to 5 mm.

20. A process for selectively removing $\beta_2$-microglobulin from blood or blood plasma comprising contacting blood or blood plasma with a porous adsorbent according to any one of claims 1 to 19.

# F I G . 1

Pore size distribution diagram

LOG(MW)

# F I G . 2

Pore size distribution diagram

LOG(MW)

# F I G. 3

**1** CARTRIDGE PACKED WITH ADSORBENT

**4** PUMP

**3** PATIENT'S BLOOD PLASMA UNDERGOING DIALYSIS

**2** BATH

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| P,X | EP-A-0 319 144 (ASAHI) <br> * Page 4, lines 23-49; page 2, lines 15-29; page 7, line 36 - page 9, line 12 * <br> --- | 1,3,14-20 | B 01 J 20/26 <br> B 01 J 20/32 |
| A | US-A-4 411 832 (P. CUATRECASAS) <br> --- | | |
| D,A | EP-A-0 211 223 (MITSUI TOATSU) <br> ----- | | |

TECHNICAL FIELDS SEARCHED (Int. Cl.5)

B 01 J.
A 61 M

The present search report has been drawn up for all claims

| Place of search | Date of completion of the search | Examiner |
|---|---|---|
| THE HAGUE | 18-05-1990 | WENDLING J.P. |

EPO FORM 1503 03.82 (P0401)